(19) Europäisches Patentamt
European Patent Office
Office européen des brevets

(11) Publication number: **0 104 690**
**A2**

# (12) EUROPEAN PATENT APPLICATION

(21) Application number: 83201249.6

(22) Date of filing: 30.08.83

(51) Int. Cl.³: A 01 N 43/40
C 07 D 213/55, C 07 D 213/56
C 07 D 213/54, C 07 D 213/26
C 07 D 213/57, C 07 F 1/08
C 07 F 13/00

(30) Priority: 27.09.82 GB 8227480

(43) Date of publication of application:
04.04.84 Bulletin 84/14

(84) Designated Contracting States:
AT BE CH DE FR GB IT LI LU NL SE

(71) Applicant: SHELL INTERNATIONALE RESEARCH MAATSCHAPPIJ B.V.
Carel van Bylandtlaan 30
NL-2596 HR Den Haag(NL)

(72) Inventor: Ten Haken, Pieter
Konkelboet The Street
Eastling Nr. Faversham Kent(GB)

(72) Inventor: Webb, Shirley Beatrice
17 North Street Ashford Road
Sheldwich Faversham Kent(GB)

(74) Representative: Hunter, Keith Roger Ian et al,
4 York Road
London SE1 7NA(GB)

## (54) Fungicidally active compositions containing ethene derivatives.

(57) A fungicidal composition which comprises a carrier and, as active ingredient, a compound of the general formula (I)

$$Ar^1R^1C = CAr^2R^2 \qquad (I)$$

or an N-oxide, salt or metal salt complex thereof, wherein one of $Ar^1$ and $Ar^2$ represents an optionally substituted 6 membered heteroaromatic ring containing 1 or 2 nitrogen atoms and the other of $Ar^1$ and $Ar^2$ represents an optionally substituted 5 or 6 membered heterocyclic ring having 1 or 2 heteroatoms or an optionally substituted phenyl group; $R^1$ represents a hydrogen atom or an optionally substituted alkyl group; and $R^2$ represents a cyano group, a -COOH group or an ester or thioester thereof, or a group

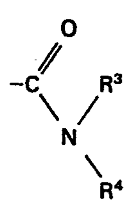 or 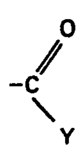

wherein each of $R^3$ and $R^4$ independently represents a hydrogen atom or an optionally substituted alkyl or phenyl group or $R^3$ and $R^4$ together with the interjacent nitrogen atom represent a heterocyclic ring and Y represents a halogen atom.

$$-OR^6,\ -SR^6,\ -\overset{\overset{O}{\|}}{S}-R^6,\ \underset{\underset{O}{\|}}{\overset{\overset{O}{\|}}{S}}-R^6,\ -OR^7,\ -SR^7,\ -CONR^8R^9 \text{ or } -NR^8R^9$$

wherein $R^6$ represents an optionally substituted alkyl or phenyl group, $R^7$ represents an acyl group derived from a carboxylic acid, and $R^8$ and $R^9$ are as defined above for $R^4$ and $R^5$ exhibit fungicidal properties.

EP 0 104 690 A2

K 1863

# FUNGICIDALLY ACTIVE COMPOSITIONS CONTAINING ETHENE DERIVATIVES

The present invention relates to fungicidally active compositions containing ethene derivatives, to novel ethene derivatives, to a process for their preparation and to a method of controlling fungal growth using the compounds and compositions.

J. Org. Chem. 20 987-9, and J. Med. Chem. 8 583-8, disclose that certain ethene derivatives may be used as intermediates in the preparation of compounds which are physiologically active. J. Org. Chem. 27 553-6 identifies further similar ethene derivates. There is no suggestion that these compounds have any use in agriculture. It has now been unexpectedly found that certain ethene derivatives of this chemical type exhibit valuable fungicidal activity.

The present invention therefore provides a fungicidal composition which comprises a carrier and, as active ingredient, a compound of the general formula (I)

$$Ar^1R^1C = CAr^2R^2 \qquad (I)$$

or an N-oxide, salt or metal salt complex thereof, wherein one of $Ar^1$ and $Ar^2$ represents an optionally substituted 6 membered heteroaromatic ring containing 1 or 2 nitrogen atoms and the other of $Ar^1$ and $Ar^2$ represents an optionally substituted 5 or 6 membered heterocyclic ring having 1 or 2 heteroatoms or an optionally substituted phenyl group; $R^1$ represents a hydrogen atom or an optionally substituted alkyl group; and $R^2$ represents a cyano group, a -COOH group or an ester or thioester thereof, or a group

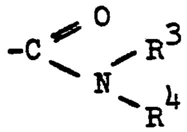 or 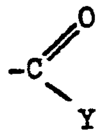

wherein each of $R^3$ and $R^4$ independently represents a hydrogen atom or an optionally substituted alkyl or phenyl group or $R^3$ and $R^4$ together with the interjacent nitrogen atom represent a heterocyclic ring and Y represents a halogen atom.

Throughout this specification, unless otherwise stated, any aliphatic moiety present preferably contains up to 6, especially up to 4, carbon atoms.

Suitable substituents which may be present in an optionally substituted aliphatic group include halogen atoms, alkoxy, alkylthio, cyano, carboxy, alkoxycarbonyl, hydroxy, amino and cycloalkyl groups and optionally substituted phenyl or phenoxy groups. Suitable substituents which may be present in an optionally substituted phenyl or phenoxy group include halogen atoms, hydroxy, methylenedioxy, amino, cyano, alkylsulphonyl and nitro groups and optionally substituted alkyl, alkenyl, alkynyl, alkoxy, alkylthio, alkoxycarbonyl, phenyl and phenoxy groups.

A phenyl group $Ar^1$ or $Ar^2$ is preferably unsubstituted or substituted by one or more of the same or different substituents given above. Most preferably it is unsubstituted, monosubstituted or disubstituted. Preferred substituents are selected from halogen atoms and nitro, cyano, alkyl, haloalkyl, alkoxy, haloalkoxy and alkylthio groups. Especially preferred substituents are 1 or 2 halogen atoms.

As noted above one or both of $Ar^1$ and $Ar^2$ represents an optionally substituted 6 membered heteroaromatic ring having 1 or 2 nitrogen atoms. These groups are optionally substituted pyridyl, pyrazinyl, pyridazinyl and pyrimidinyl groups.

The other of $Ar^1$ and $Ar^2$ may represent an optionally substituted 5 or 6 membered heterocyclic ring having 1 or 2 heteroatoms. Suitable heteroatoms are nitrogen, oxygen and sulphur; suitable groups include those listed above and also thiophene,

furan, pyrrole, pyrazole, pyrrolidine, imidazole and the corresponding partially or fully saturated analogues. Thiophene and furan groups are especially suitable.

A heterocyclic group $Ar^1$ or $Ar^2$ may be substituted by one or more of the substituents given above for a phenyl group; preferably however it is unsubstituted or substituted by one or more alkyl groups or halogen atoms; most preferably such a group is unsubstituted.

As stated above, the invention includes compositions containing metal salt complexes and salts of compounds of the general formula I. Suitable salts include salts with sulphonic acids, for example benzene- or toluenesulphonic acid, carboxylic acids for example tartaric or acetic acid, or inorganic acids for example the hydrohalic acids or sulphuric acid. If $R^2$ represents a -COOH group, a salt may be a metal salt, for example an alkali or alkaline earth metal salt, the ammonium salt, or a substituted ammonium salt, for example an alkyl-substituted ammonium salt. Suitable metal salts which form complexes with compounds of the general formula I include those of heavy metals such as iron, copper, zinc and manganese, in which the anions may, for example, be derived from one of those acids given above.

The compounds of the general formula I exist as geometric isomers. Optical isomers may be present, the number of possibilities for isomerism depending on the specific groups present. The general formula I should be understood to include all individual isomers and mixtures thereof.

Prefered compounds of the general formula I and N-oxides, salts and complexes thereof, are those in which one of $Ar^1$ and $Ar^2$ represents an unsubstituted pyrazine or, especially, an unsubstituted pyridyl group and the other of $Ar^1$ and $Ar^2$ represents an unsubstituted pyridyl group or an optionally substituted phenyl group; more preferably one of $Ar^1$ and $Ar^2$ is an unsubstituted 3-pyridyl group and the other of $Ar^1$ and $Ar^2$ is an unsubstituted 3-pyridyl group or a halo-substituted phenyl group for

example a mono or di-chloro or fluoro substituted phenyl group, preferably a dichloro substituted phenyl group.

Preferably $R^1$ represents a methyl group or, especially, a hydrogen atom.

If $R^2$ represents an ester or thioester of -COOH, this ester or thioester may for example be derived from an optionally substituted alkyl or phenyl alcohol or thiol. Preferred substituents are, for example, as given above for alkyl and phenyl groups. Especially preferred are unsubstituted alkanols and alkane thiols having up to 8, especially up to 6, carbon atoms.

If $R^3$ and/or $R^4$ represent an optionally substituted alkyl or phenyl group, the preferred optional substituents are, for example, those given above. For example, a phenyl group $R^3$ and/or $R^4$ may be substituted as described above for $Ar^1$ or $Ar^2$.

If $R^3$ and $R^4$ together with the interjacent nitrogen atom represent a heterocyclic ring, this ring may be saturated or unsaturated, and preferably contains 5 or 6 atoms which may include heteroatoms, for example an oxygen or a nitrogen atom, in addition to the interjacent nitrogen atom.

Preferably $R^2$ represents a cyano group, a -COOH group or an ester or thio ester thereof, or a group

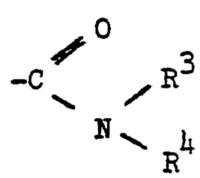

or

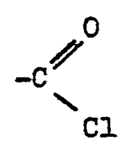

wherein each of $R^3$ and $R^4$ independently represents a hydrogen atom, an unsubstituted alkyl group of up to 6 carbon atoms, or an optionally substituted phenyl group, or $R^3$ and $R^4$ together with the interjacent nitrogen atom represent a pyrrolidine or piperidine ring.

Most of the compounds are believed to be novel. The invention therefore also provides a compound of the general formula I or an N-oxide, salt or metal salt complex thereof, with the proviso that when $Ar^1$ is an unsubstituted pyridyl group and $R^1$ is

a hydrogen atom, $R^2$ is not a cyano group when $Ar^2$ is an unsubstituted phenyl group or a 2-chloro, 4-chloro, 4-amino, 4-dimethylamino, 4-acetamido, 4-nitro, 4-methoxy or 3,4-dimethoxy substituted phenyl group, $R^2$ is not a $-CONH_2$ group when $Ar^2$ is an unsubstituted phenyl group or a 4-chloro or 4-dimethylamino group, $R^2$ is not a -COOH group when $Ar^2$ is an unsubstituted phenyl group or a 4-nitrophenyl group, and $R^2$ is not a -COCl group when $Ar^2$ is an unsubstituted phenyl group; and further provided that if $Ar^2$ is an unsubstituted pyridyl group and $R^1$ is a hydrogen atom, $R^2$ is not a cyano group if $Ar^1$ is an unsubsituted phenyl group or a 4-dimethylaminophenyl group, and $R^2$ is not a -COOH, -COCl or $-CONH_2$ group if $Ar^1$ is an unsubstituted phenyl group.

The invention further provides a process for the preparation of a novel compound according to the invention which comprises reacting a compound of the general formula (II)

$$Ar^1-\overset{O}{\overset{\|}{C}}-R^1 \qquad (II)$$

with a compound of the general formula III

$$Ar^2 - CH_2 - R^2 \qquad (III)$$

wherein $Ar^1$, $Ar^2$, $R^1$ and $R^2$ have the meanings given for the novel compounds of the general formula I; and if desired, converting the resulting compound of the invention into any other compound of the invention.

In general, the process according to the invention may lead to a mixture of geometric isomers of the general formula I. When $R^2$ represents a cyano group the predominant isomer formed is usually that in which $Ar^1$ and $Ar^2$ are <u>trans</u>, whereas when $R^2$ is other than cyano the corresponding <u>cis</u> isomer usually predominates. The exact ratio of products produced depends of course on the precise reaction conditions.

The molar ratio of the reactants is not critical and may for example be in the range of from 5:1 to 1:5, especially 2:1 to 1:2. It is often convenient to use approximately stoichiometric

ratios.

The reaction is suitably carried out in the presence of a solvent; typical solvents include, for example, alcohols such as methanol or ethanol; ethers such as dimethoxyethane or tetrahydrofuran; chlorinated hydrocarbons such as methylene chloride; anhydrides such as acetic anhydride; esters such as ethyl acetate; amides such as dimethyl formamide or dimethyl acetamide; ketones such as acetone, dimethyl ketone and methyl ether ketone; and nitroalkanes such as nitromethane.

The reaction is preferably carried out in the presence of a base. Suitable bases include primary, secondary or tertiary amines, for example, triethylamine or piperidine; alkali metal hydrides, amides or alkoxides, for example, sodium ethoxide; or alkali metal or alkaline earth metal hydroxides, for example, potassium hydroxide. The reaction temperature is suitably in the range of from $0^{\circ}$ to $180^{\circ}C$. It may in some cases be convenient to carry out the reaction at the reflux temperature of the reaction mixture.

Compounds of the formula II and III may be prepared by methods analogous to methods known in the art.

As stated above, a resulting compound of the invention may, if desired, be converted into any other compound of the invention. Such reactions may be carried out by methods analogous to methods known in the art. For example, a compound in which $R^2$ represents a cyano group may be hydrolysed or alcoholised to give the appropriate acid, ester or amide. A compound in which $R^2$ represents a -COOH group may be converted into an ester or thioester by reaction with the appropriate alcohol or thiol, or into the acid halide by reaction with a halogenating agent. An acid halide may also be converted into an ester or thioester by reaction with the appropriate alcohol or thiol or may be converted into an amide with the appropriate amine.

For practical reasons, for example availability of starting materials, it may often be preferred to use as reactant a compound of the formula III in which $R^2$ represents a -CN or -COOH

group, and subsequently to convert the resulting compound into the desired derivative. This method may also be used to prepare a different isomer where the initial reaction of the compounds II and III gives a large predominance of one isomer. For example, if, as explained above, a reaction using a compound of formula III in which $R^2$ represents a -COOH group leads largely to a product in which $Ar^1$ and $Ar^2$ are cis to each other, while the corresponding reaction in which $R^2$ represents a -CN group gives largely the trans isomer, the trans isomer of a product in which $R^2$ is a -COOH group is conveniently prepared by hydrolysis of the trans -CN compound.

A compound of formula I may be converted into its N-oxide or a salt or a metal salt complex thereof by methods analogous to known methods, for example by reaction with an oxidising agent or with the appropriate acid, base or salt. A resulting salt can be converted into the free compound by reaction with an acid binding agent or an acid, as appropriate.

The invention further provides a method of controlling fungus at a locus, which comprises applying to the locus a compound or a composition according to the invention. Suitable dosages are, for example, in the range of from 0.05 to 4kg active material per hectare. The method of the invention is especially useful for the treatment or prevention of fungal attack in seeds, soil or plants; crops susceptible to powdery mildews, for example cereals or apples, may for example be treated.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating fungicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montomorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene

oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing ½-10% w of active ingredient. Granules are usually prepared to have a size between 10 and 100 BS mesh (1.676 - 0.152 mm), and may be manufactured by agglomeration or impregnation techniques. Generally, granules will contain ½-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants

and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'-like consistency.

The composition of the invention may also contain other ingredients, for example, other compounds possessing herbicidal, insecticidal or fungicidal properties.

The following Examples illustrate the invention. The terms "cis" and "trans" are used to indicate the relationship of $Ar^1$ and $Ar^2$ about the double bond.

Example 1

Preparation of 2-(2,4-dichlorophenyl)-3-(3-pyridyl)-propenoic acid (cis isomer)

A mixture of pyridine-3-aldehyde (21.4g), 2,4-dichlorophenylacetic acid (40.1g), triethylamine (20ml) and acetic anhydride (160ml) was stirred and heated under reflux for 5 hours while maintaining a temperature of 150°C. After cooling, the reaction mixture was diluted with water (150ml) and left to stand overnight. The crystalline solid that had separated was filtered off, washed thoroughly with water and dried. After recrystallisation from ethanol (600ml) the cis 2-(2,4-dichlorophenyl)-3-(3-pyridyl)-propenoic acid was obtained as pale yellow crystals; melting point 208-211°C; yield 71%. The following elemental

analysis results were obtained:

| | | | |
|---|---|---|---|
| Calculated: | C: 57.14 | H: 3.06 | N: 4.76 |
| Found: | C: 57.1 | H: 3.2 | N: 4.7 |

Example 2

Preparation of 2-(2,4-dichlorophenyl)-3-(3-pyridyl)-propenyl chloride hydrochloride (cis isomer)

A mixture of cis 2-(2,4-dichlorophenyl)-3-(3-pyridyl)-propenoic acid (32.1g) and thionyl chloride (150ml) was stirred and heated under reflux for 3 hours. Excess thionyl chloride was removed in vacuo and the residue triturated with dry diethyl ether. The solid acid chloride hydrochloride was filtered off, washed with diethyl ether and dried in a vacuum oven. The yield of product was 82%.

The following elemental analysis results were obtained:-

| | | | |
|---|---|---|---|
| Calculated: | C: 48.14 | H: 2.58 | N: 4.01 |
| Found | C: 47.8 | H: 2.6 | N: 4.0 |

Example 3

Preparation of n-butyl 2-(2,4-dichlorophenyl)-3-(3-pyridyl)-propenoic acid ester (cis isomer)

Sodium (0.9g) was dissolved in dry n-butanol (70ml) under nitrogen, and the resultant solution added to a stirred suspension of 2-(2,4-dichlorophenyl)-3-(3-pyridyl)-propenoyl chloride hydrochloride (6.8g) in dry dimethoxyethane. The mixture was stirred and heated under reflux for 16 hours. After cooling, the mixture was filtered and the solvent evaporated from the filtrate under reduced pressure. The residue was taken up in methylene chloride, washed three times with water and dried using magnesium sulphate.

After filtration and removal of the solvent in vacuo, the residual oil was subjected to column chromatography on silica gel, eluting with diethyl ether/hexane (2:1). The n-butyl 2-(2,4-dichlorophenyl)-3-(3-pyridyl)-propenoic acid ester was obtained in 75% yield as a pale yellow oil.

The following elemental analysis results were obtained:-

| | | | |
|---|---|---|---|
| Calculated: | C: 61.71 | H: 4.86 | N: 4.00 |
| Found: | C: 62.2 | H: 5.1 | N: 3.9 |

Example 4

Preparation of (1-methylpropyl) 2-(2,4-dichlorophenyl)-3-(3-pyridyl)-thiolo-propenoic acid ester (cis isomer)

A stirred suspension 2-(2,4-dichlorophenyl)-3-(3-pyridyl)-propenoyl chloride hydrochloride (6.35g) in dry dimethoxyethane (50ml) under nitrogen was treated with dry triethylamine (1.85g). A suspension of the sodium salt of 1-methylpropane-1-thiol (0.0182 mole) in dry dimethoxyethane (25ml) was then added, and the mixture stirred and heated under reflux for 16 hours. After cooling, the mixture was filtered and solvent recovered from the filtrate *in vacuo*. The residual oil was taken up in diethyl ether, washed three times with water and dried using magnesium sulphate. After filtration and removal of the solvent, the residual oil was subjected to column chromatography on silica gel, eluting with diethyl ether/hexane (2:1). The (1-methylpropyl) 2-(2,4-dichlorophenyl)-3-(3-pyridyl)-thiolo-propenoic acid ester was thus obtained in 52% yield as a pale yellow oil.

The following elemental analysis results were obtained:-

| | | | |
|---|---|---|---|
| Calculated: | C: 59.02 | H: 4.64 | N: 3.83 |
| Found | C: 58.5 | H: 4.7 | N: 3.8 |

Example 5

Preparation of 1-(N-piperidinylcarbonyl)-1-(2,4-dichlorophenyl)-2-(3-pyridyl)ethene.

To a stirred suspension of 3-(3-pyridyl)-2-(2,4-dichlorophenyl)-propenoyl chloride hydrochloride (6.23g) in dry dimethoxyethane (50ml), cooled in an ice-bath to 5°C, was added dropwise a solution of piperidine (4.55g) in dry dimethoxyethane (25ml). The mixture was stirred at room temperature for 20 hours. After filtration, solvent was recovered from the filtrate *in vacuo*, and

the residue was taken up in methylene chloride, washed three times with water and dried using magnesium sulphate. After filtration and removal of solvent *in vacuo*, the residue was subjected to column chromatography on silica gel, eluting with diethyl ether to give a 47% yield of the desired compound as very pale yellow crystals; mpt. 102-104°C.

The following elemental analysis results were obtained:-

| | | | |
|---|---|---|---|
| Calculated: | C: 63.16 | H: 4.99 | N: 7.76 |
| Found: | C: 62.9 | H: 5.1 | N: 7.7 |

Example 6

Preparation of 1-cyano-1-(4-chlorophenyl)-2-(3-pyridyl)ethene (trans isomer)

A solution of 4-chlorobenzyl cyanide (7.58g) and pyridine-3-aldehyde (5.35g) in absolute ethanol (50ml) was warmed to 50°C; 3.5ml of a solution of sodium (2.74g) in absolute ethanol (32ml) was added and the mixture was left without further heating. After 1 hour, the solid product was filtered off, washed with ethanol, and then diethylether, and dried. Recrystallisation of this material from ethanol (150ml) with charcoal treatment gave the 1-cyano-1-(4-chlorophenyl)-2-(3-pyridyl)ethene in 48% yield as pale yellow needles; mpt. 141-143°C

The following elemental analysis results were obtained:-

| | | | |
|---|---|---|---|
| Calculated: | C: 69.85 | H: 3.74 | N: 11.64 |
| Found: | C: 69.9 | H: 3.7 | N: 11.6 |

Example 7

Preparation of the copper (II) chloride complex of the compound of Example 6

To a stirred solution of 1-cyano-1-(4-chlorophenyl)-2-(3-pyridyl)ethene (2.4g) in warm ethanol (50ml) was added a solution of copper (II) chloride (0.6725g) in ethanol (15ml). After ½ hour the pale blue green copper (II) chloride complex (2 moles of ethene derivative per mole of $CuCl_2$) was filtered off, washed with ethanol, then diethylether, and dried. The yield was 89%, mpt. 290-292°C (decomposition).

Elemental Analysis:

| | | | |
|---|---|---|---|
| Calculated: | C: 54.59 | H: 2.92 | N: 9.1 |
| Found: | C: 53.8 | H: 2.7 | N: 9.0 |

Examples 8 to 38

By methods analogous to those described in Examples 1 to 7, the following compounds were prepared.

Analysis and physical data figures where available are given in Table I.

TABLE I

| Example No. (cis/trans isomer) | $Ar^1$ | $Ar^2$ | $R^1$ | $R^2$ | | Elemental Analysis C | H | N | Physical data |
|---|---|---|---|---|---|---|---|---|---|
| 8 (cis) | 2,4-dichloro-phenyl | 3-pyridyl | H | COOH | Calc: | 57.40 | 3.06 | 4.76 | Mpt: 262-264°C |
| | | | | | Found: | 56.6 | 3.1 | 4.5 | |
| 9 (cis) | 3-pyridyl | 2,4-dichloro-phenyl | H | $-CON$ $(CH_2)_3CH_3$<br>$(CH_2)_3CH_3$ | Calc: | 65.19 | 6.42 | 6.91 | oil |
| | | | | | | 65.2 | 6.9 | 6.8 | |
| 10 (trans) | 4-F-phenyl | 3-pyridyl | H | CN | Calc: | 75.00 | 4.00 | 12.50 | Mpt: 145-147°C |
| | | | | | Found: | 75.6 | 4.1 | 12.5 | |
| 11 (trans) | 4-Cl-phenyl | 3-pyridyl | H | CN | Calc: | 69.85 | 3.74 | 11.64 | Mpt: 112-114°C |
| | | | | | Found: | 70.3 | 3.7 | 11.6 | |

TABLE I (Ctd.)

| Example No. (cis/trans isomer) | $Ar^1$ | $Ar^2$ | $R^1$ | $R^2$ | | Elemental Analysis C | H | N | Physical data |
|---|---|---|---|---|---|---|---|---|---|
| 12 (trans) | 3-pyridyl | 2,4-dichloro-phenyl | H | CN | Calc: | 61.1 | 2.91 | 10.18 | Mpt: 114-116°C |
| | | | | | Found: | 60.9 | 2.8 | 9.9 | |
| 13 (cis) | 3-pyridyl | 4-Cl-phenyl | H | COOH | Calc: | 64.74 | 3.85 | 5.39 | Mpt: 221-224°C |
| | | | | | Found: | 64.7 | 3.9 | 5.4 | |
| 14 (cis) HCl Salt | 2,4-dichloro-phenyl | 3-pyridyl | H | COCl | Calc: | 48.14 | 2.58 | 4.01 | |
| | | | | | Found: | 46.4 | 2.9 | 4.0 | |
| 15 (cis) | 2,4-dichloro-phenyl | 3-pyridyl | H | $CH_3$ COOCH $CH_2CH_3$ | Calc: | 61.71 | 4.00 | 4.00 | Oil |
| | | | | | Found: | 61.5 | 4.8 | 3.9 | |
| 16 (cis) | 2,4-dichloro-phenyl | 3-pyridyl | H | CON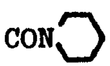 | Calc: | 63.16 | 4.99 | 7.76 | Mpt: 157-158°C |
| | | | | | Found: | 63.4 | 5.2 | 7.7 | |

TABLE I (Ctd.)

| Example No. (cis/trans isomer) | $Ar^1$ | $Ar^2$ | $R^1$ | $R^2$ | Elemental Analysis | C | H | N | Physical data |
|---|---|---|---|---|---|---|---|---|---|
| 17 (cis) | 3-pyridyl | 2,4-dichloro-phenyl | H | $CONHC(CH_3)_3$ | Calc: | 61.89 | 5.16 | 8.02 | Mpt: 116-118°C |
| | | | | | Found: | 61.9 | 5.3 | 8.1 | |
| 18 (cis) | 2,4-dichloro-phenyl | 3-pyridyl | H | $COSC(CH_3)_3$ | Calc: | 59.02 | 4.64 | 3.83 | Mpt: 87-89°C |
| | | | | | Found: | 59.2 | 4.8 | 3.8 | |
| 19 (cis) | 2,4-dichloro-phenyl | 3-pyridyl | H | $COOCH_3$ | Calc: | 58.44 | 3.57 | 4.55 | Oil |
| | | | | | Found: | 58.3 | 3.7 | 4.5 | |
| 20 (cis) | 2,4-dichloro-phenyl | 3-pyridyl | H | $COO(CH_2)_3CH_3$ | Calc: | 61.71 | 4.86 | 4.00 | Oil |
| | | | | | Found: | 61.9 | 5.3 | 4.0 | |
| 21 (trans) | 3-pyridyl | 2,4-dichloro-phenyl | H | $COOCH_3$ | Calc: | 58.44 | 3.57 | 4.55 | Oil |
| | | | | | Found: | | | | |
| 22 (cis) | 2,4-dichloro-phenyl | 3-pyridyl | H | $COOCH_2CH_3$ | Calc: | 59.63 | 4.04 | 4.35 | Mpt: 64-68°C |
| | | | | | Found: | 59.6 | 4.2 | 4.5 | |

TABLE I (Ctd.)

| Example No. (cis/trans isomer) | $Ar^1$ | $Ar^2$ | $R^1$ | $R^2$ | | Elemental Analysis C | H | N | Physical data |
|---|---|---|---|---|---|---|---|---|---|
| 23 (trans) Tartaric acid salt (2 moles tartaric acid per mole pyridine compound) | 3-pyridyl | 2,4-dichloro-phenyl | H | 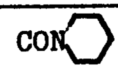 | Calc:<br>Found: | 49.02<br>49:2 | 4.54<br>4.6 | 4.24<br>4.3 | Mpt: 124-125°C |
| 24 (cis) | 3-pyridyl | 2,4-dichloro-phenyl | H | $COOCH_3$ | Calc:<br>Found: | 58.44<br>58.80 | 3.57<br>3.90 | 4.55<br>4.50 | Mpt: 41-47°C |
| 25 (cis) | 3-pyridyl | 2,4-dichloro-phenyl | H | 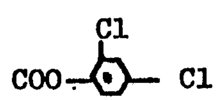 | Calc:<br>Found: | 54.67<br>54.7 | 2.51<br>2.5 | 3.19<br>3.1 | glass |
| 26 (cis) | 3-pyridyl | 2,4-dichloro-phenyl | H | 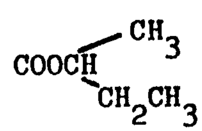 | Calc:<br>Found: | 61.71<br>61.7 | 4.86<br>4.9 | 4.00<br>4.0 | oil |
| 27 (cis) | 3-pyridyl | 2,4-dichloro-phenyl | H | 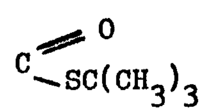 | Calc:<br>Found: | 59.02<br>59.0 | 4.64<br>5.1 | 3.83<br>3.8 | Oil |

TABLE I (Ctd.)

| Example No. (cis/trans isomer) | $Ar^1$ | $Ar^2$ | $R^1$ | $R^2$ | | Elemental Analysis C | H | N | Physical data |
|---|---|---|---|---|---|---|---|---|---|
| 28 (cis) | 3-pyridyl | 2,4-dichloro-phenyl | H | 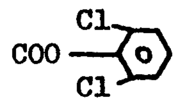 | Calc: | 66.33 | 4.27 | 3.32 | Oil |
| | | | | | Found: | 66.3 | 4.1 | 3.2 | |
| 29 (cis) | 3-pyridyl | 2,4-dichloro-phenyl | H | 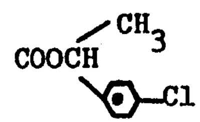 | Calc. | 61.04 | 3.70 | 3.24 | Oil |
| | | | | | Found: | 61.3 | 3.9 | 3.2 | |
| 30 (cis) | 3-pyridyl | phenyl | H | $COOCH_2CH_3$ | Calc: | 75.89 | 5.93 | 5.53 | Mpt: 67-68°C |
| | | | | | Found: | 75.9 | 6.1 | 5.5 | |
| 31 (cis) copper (II) chloride complex (2 moles pyridine compound per mole $CuCl_2$) | 3-pyridyl | 2,4-dichloro-phenyl | H | 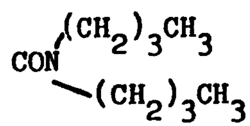 | Calc: | 55.90 | 5.51 | 5.93 | Mpt: 169-170°C |
| | | | | | Found: | 55.6 | 5.5 | 6.0 | (decomposition) |

TABLE I (Ctd.)

| Example No. (cis/trans isomer) | $Ar^1$ | $Ar^2$ | $R^1$ | $R^2$ | | Elemental Analysis C | H | N | Physical data |
|---|---|---|---|---|---|---|---|---|---|
| 32 (cis) copper (II) chloride complex (2 moles pyridine compound per mole $CuCl_2$) | 3-pyridyl | 2,4-dichloro-phenyl | H | $CONHC(CH_3)_3$ | Calc.<br>Found: | 51.89<br>51.7 | 4.32<br>4.6 | 6.73<br>6.5 | Mpt: 127-130°C (decomposition) |
| 33 (cis) copper (II) chloride complex (2 moles pyridine compound per mole $CuCl_2$) | 3-pyridyl | 2,4-dichloro-phenyl | H | $COOCH_3$ | Calc:<br>Found: | 47.97<br>46.5 | 2.93<br>3.1 | 3.73<br>3.5 | Mpt: 176-178°C (decomposition) |
| 34 (trans) | 3-pyridyl | 2,4-dichloro-phenyl | H | $(CH_2)_3CH_3$<br>CON<br>$(CH_2)_3CH_3$ | Calc:<br>Found: | 65.19<br>65.4 | 6.42<br>6.7 | 6.91<br>7.0 | Mpt: 61-62°C |

TABLE I (Ctd.)

| Example No. (cis/trans isomer) | $Ar^1$ | $Ar^2$ | $R^1$ | $R^2$ | | Elemental Analysis C | H | N | Physical data |
|---|---|---|---|---|---|---|---|---|---|
| 35 (cis) | 3-pyridyl | 3-pyridyl | H | COOH | Calc. | 69.03 | 4.42 | 12.39 | Mpt: 200-201°C |
| | | | | | Found. | 68.1 | 4.8 | 11.6 | |
| 36 (cis) HCl Salt (2 moles HCl per mole ethene derivative) | 3-pyridyl | 3-pyridyl | H | COCl | Calc. | 49.13 | 3.46 | 8.82 | |
| | | | | | Found. | 50.3 | 5.6 | 8.7 | |
| 37 (cis) | 3-pyridyl | 3-pyridyl | H | $COOCH(CH_3)-CH_2CH_3$ | Calc. | 72.34 | 6.38 | 9.93 | Mpt. 53-55°C |
| | | | | | Found. | 72.5 | 6.4 | 10.0 | |
| 38 (cis) | 3-pyridyl | 3-pyridyl | H | $COOCH_3$ | Calc. | 70.0 | 5.0 | 11.67 | Mpt. 100-103°C |
| | | | | | Found. | 69.8 | 5.0 | 11.6 | |

TABLE I (Ctd.)

| Example No. (cis/trans isomer) | $Ar^1$ | $Ar^2$ | $R^1$ | $R^2$ | Elemental Analysis | C | H | N | Physical data |
|---|---|---|---|---|---|---|---|---|---|
| 39 (trans) | 3-pyridyl | 2,4-$Cl_2$-phenyl | CN | H | Calc. | 61.1 | 2.91 | 10.18 | Mpt: 148-151°C |
| | | | | | Found | 61.1 | 2.7 | 10.2 | |
| 40 (cis) | 3-pyridyl | 2,4-$Cl_2$-phenyl | CN | $CH_3$ | Calc. | 62.28 | 3.46 | 9.69 | |
| | | | | | Found | 62.6 | 3.6 | 9.5 | |
| 41 (trans) | 3-pyridyl | 2,4-$Cl_2$-phenyl | CN | $CH_3$ | Calc. | 62.28 | 3.46 | 9.96 | |
| | | | | | Found | 62.2 | 3.4 | 9.7 | Mpt: 131-133°C |
| 42 (trans) | 3-pyridyl | 2,4-$Cl_2$-phenyl | COOH | H | Calc. | 57.4 | 3.06 | 4.76 | Mpt: 185-190°C |
| | | | | | Found | 57.4 | 3.0 | 4.9 | |
| 43 (trans) | 3-pyridyl | 2,4-$Cl_2$-phenyl | H | $CONH_2$ | Calc. | 57.34 | 3.41 | 9.56 | Mpt: 171-173°C |
| | | | | | Found | 57.1 | 3.2 | 9.2 | |
| 44 (trans) | 3-pyridyl | 2,4-$Cl_2$-phenyl | $CONH_2$ | H | Calc. | 57.34 | 3.41 | 9.56 | Mpt: 222-223°C |
| | | | | | Found | 57.3 | 3.2 | 9.4 | |
| 45 (cis) $CuCl_2$ complex | 3-pyridyl | 2,4-$Cl_2$-phenyl | $COOCH_3$ | H | Calc. | 47.97 | 2.93 | 3.73 | Mpt: 162-164°C |
| | | | | | Found | 47.5 | 2.8 | 3.4 | |
| 46 (trans) | 3-pyridyl | 2,4-$Cl_2$-phenyl | H | H | Calc. | 62.4 | 3.6 | 5.6 | Mpt: 86-89°C |
| | | | | | Found | 62.0 | 3.5 | 5.5 | |

TABLE I (Ctd.)

| Example No. (cis/trans isomer) | $Ar^1$ | $Ar^2$ | $R^1$ | $R^2$ | | Elemental Analysis C | H | N | Physical data |
|---|---|---|---|---|---|---|---|---|---|
| 47 (cis) | 3-pyridyl | 2,4-$Cl_2$-phenyl | H | CN | Calc.<br>Found | 61.1<br>61.1 | 2.91<br>2.9 | 10.18<br>9.9 | Mpt: 66-67.5°C |
| 48 (cis) | 2,4-$Cl_2$-phenyl | 3-pyridyl | H | $CONHC(CH_3)_3$ | Calc.<br>Found | 61.89<br>61.5 | 5.16<br>5.2 | 8.02<br>7.9 | Mpt: 114-116°C |
| 49 (trans) $CuCl_2$ complex | 3-pyridyl | 2,4-$Cl_2$-phenyl | CN | H | Calc.<br>Found | 49.09<br>48.5 | 2.34<br>2.5 | 8.18<br>8.0 | Mpt: 305-307°C |
| 50 (cis) | 2,4-$Cl_2$-phenyl | 3-pyridyl | H | $CONHCH_2CH_2OH$ | Calc.<br>Found | 56.97<br>56.3 | 4.15<br>4.1 | 8.31<br>7.7 | Mpt: 120-124°C |
| 51 (cis) | 2,4-$Cl_2$-phenyl | 3-pyridyl | H | $CONHCH_2\overset{\overset{O}{\|}}{C}OEt$ | Calc.<br>Found | 56.99<br>57.2 | 4.22<br>4.1 | 7.39<br>7.1 | Mpt: 101-103°C |
| 52 (trans) | 2,4-$Cl_2$-phenyl | 3-pyridyl | H | $CONHC(CH_3)_3$ | Calc.<br>Found | 61.89<br>60.2 | 5.16<br>5.2 | 8.02<br>7.5 | Mpt: 181-186°C |
| 53 (cis) | 2,4-$Cl_2$-phenyl | 3-pyridyl | H | $CONH(CH_2)_2O\overset{\overset{O}{\|}}{C}CH_3$ | Calc.<br>Found | 56.99<br>56.8 | 4.22<br>4.4 | 7.39<br>7.4 | Mpt: 105-106°C |

TABLE I (Ctd.)

| Example No. (cis/trans isomer) | $Ar^1$ | $Ar^2$ | $R^1$ | $R^2$ | Elemental Analysis | C | H | N | Physical data |
|---|---|---|---|---|---|---|---|---|---|
| 54 (not determined) | 3-pyridyl | 2,4-$Cl_2$-phenyl | OH | CN | Calc.<br>Found | 57.73<br>57.8 | 2.75<br>2.7 | 9.62<br>9.6 | Mpt: 189-191°C |
| 55 (cis) | 2,4-$Cl_2$-phenyl | 3-pyridyl | H | 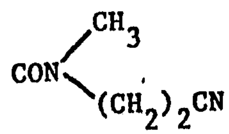 | Calc.<br>Found | 60.00<br>59.7 | 4.17<br>4.1 | 11.50<br>11.4 | Mpt: 90-92°C |
| 56 (trans) | 2,4-$Cl_2$-phenyl | 3-pyridyl | H | $CONH(CH_2)_2O\overset{O}{\overset{\|}{C}}CH_3$ | Calc.<br>Found | 56.99<br>56.9 | 4.22<br>4.2 | 7.39<br>7.2 | Mpt: 102-104°C |
| 57 (trans) | 2,4-$Cl_2$-phenyl | 3-pyridyl | H | 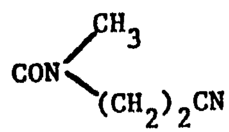 | Calc.<br>Found | 60.0<br>59.3 | 4.17<br>3.9 | 11.67<br>11.5 | Mpt: 85-87°C |
| 58 (not determined) | 3-pyridyl | 2-OEt,4-Cl-phenyl | CN | OH | Calc.<br>Found | 63.89<br>63.6 | 4.33<br>4.2 | 9.32<br>9.0 | Mpt: 235°C |
| 59 (trans) $MnCl_2$ complex | 3-pyridyl | 2,4-$Cl_2$-phenyl | CN | $CH_3$ | Calc.<br>Found | 51.14<br>51.2 | 2.84<br>2.7 | 7.96<br>7.8 | Mpt: 242-244°C |
| 60 (cis) $MnCl_2$ complex | 3-pyridyl | 2,4-$Cl_2$-phenyl | CN | $CH_3$ | Calc.<br>Found | 51.14<br>51.6 | 2.84<br>2.9 | 7.96<br>7.9 | Mpt: 245-248°C |

Example 39

(a) Activity against vine downy mildew (Plasmopara viticola; P.v.a.)

The test is a direct antisporulant one using a foliar spray. The lower surfaces of leaves of whole vine plants are inoculated by spraying with an aqueous suspension containing $10^5$ zoosporangia/ml 2 days prior to treatment with the test compound. The inoculated plants are kept for 24 hours in a high humidity compartment, and then 24 hours at glasshouse ambient temperature and humidity. The plants are then dried and infected leaves detached and sprayed on the lower surfaces with a solution of active material in 1:1 water/acetone containing 0.04% Triton X-155 (Trade Mark). The spraying is carried out with a moving track sprayer which delivers 620 l/ha, and the concentration of active material is calculated to give an application rate of lkg/ha. After drying, the petioles of the sprayed leaves are dipped in water and the leaves returned to high humidity for a further 96 hours incubation, followed by assessment. Assessment is based on the percentage of the leaf area covered by sporulation compared with that on control leaves.

(b) Activity against vine downy mildew (Plasmopara viticola; Pv.t)

The test is a translaminar protectant one using a foliar spray. The upper surfaces of leaves of whole vine plants are sprayed at a dosage of 1 kilogram of active material per hectare using a track sprayer. The lower surfaces of the leaves are then inoculated, up to 6 hours after treatment with the test compound, by spraying with an aqueous suspension containing $10^5$ zoosporangia/ml. The inoculated plants are kept for 24 hours in a high humidity compartment, 4 days at glasshouse ambient temperature and humidity and then returned for a further 24 hours to high humidity. Assessment is based on the percentage of leaf area covered by sporulation compared with that on control leaves.

(c) Activity against vine grey mould (Botrytis cinerea; B.c.)

The test is a direct eradicant one using a foliar spray. The under-surface of the detached vine leaves are inoculated by pipetting ten large drops of an aqueous suspension containing $5x10^5$ conidia/ml on to them. The inoculated leaves are kept uncovered overnight during which time the fungus has penetrated the leaf and a visible necrotic lesion may be apparent where the drop was made. The infected regions are sprayed directly with a dosage of 1kg of active material per hectare using a track sprayer as described under (a). When the spray has dried the leaves are covered with a petri dish and the disease allowed to develop under these humid conditions. The extent of the necrotic lesion beyond the original drop together with the degree of sporulation is compared with that on control leaves.

(d) Activity against potato late blight (Phytophtora infestans; P.i.p.)

The test measures the direct protectant activity of compounds applied as a foliar spray. Tomato plants, cultivar Ailsa Craig, 1-15cms high, in monopots are used. The whole plant is sprayed at a dosage of 1 kilogram of active material per hectare using a track sprayer. The plant is then inoculated up to 6 hours after treatment with the test compound, by spraying with an aqueous suspension containing $5 \times 10^3$ zoosporangia/ml. The inoculated plants are kept in high humidity for 3 days. Assessment is based on comparison between the levels of disease on the treated and control plants.

(e) Activity against barley powdery mildew (Erysiphe graminis; E.g.)

The test measures the direct antisporulant activity of compounds applied as a foliar spray. For each compound about 40 barley seedlings are grown to the one-leaf stage in a plastic pot of sterile potting compost. Inoculation is effected by dusting the leaves with conidia of Erysiphe graminis, spp. hordei. 24 hours after inoculation the seedlings are sprayed

with a solution of the compound in a mixture of acetone (50%), surfactant (0.04%) and water using a track sprayer as described under (a). The rate of application is equivalent to 1kg of active material per hectare. First assessment of disease is made 5 days after treatment, when the overall level of sporulation on the treated plants is compared with that on control plants.

(f) Activity against apple powdery mildew (Podsophaera leucotrica; P.l.)

The test is a direct anti-sporulant one using a foliar spray. The upper surfaces of leaves of whole apple seedlings are inoculated by spraying with an aqueous suspension containing $10^5$ conidia/ml 2 days prior to treatment with the test compound. The inoculated plants are immediately dried and kept at glasshouse ambient temperatures and humidity prior to treatment. The plants are sprayed at a dosage of 1 kilogram of active material per hectare using a track sprayer. After drying the plants are returned to a compartment at ambient temperature and humidity for up to 9 days, followed by assessment. Assessment is based on the percentage of the leaf area covered by sporulation compared with that on leaves of control plants.

(g) Activity against peanut leaf spot (Cercospora arachidicola; Ca)

The test is a direct eradicant one using a foliar spray. The upper surfaces of the leaves of peanut plants (12-20cms high, monopots) are inoculated by spraying with an aqueous suspension containing $10^5$ conidia/ml 4 hours prior to treatment with the test compound. The inoculated plants are kept at high humidity and then allowed to dry during the interval between inoculation and treatment by spraying at a dosage of 1kg of active material per hectare using a track sprayer. After spraying the plants are moved to a humid compartment at 25-28°C for a further period of up to 10 days. Assessment is based on a comparison between the levels of disease on the treated and control plants.

The extent of disease control achieved in these tests is expressed as a control rating in Table II below; greater than 80% disease control is given the rating 2 after the test; control of between 50 and 80% is given the rating 1 after the test.

Table II

| Compound of Example No. | Greater than 50% disease control achieved in the below indicated tests |
|---|---|
| 1 | Pvt (2) Pl (2) |
| 3 | Pva (1) Eg (2) Pl (2) |
| 4 | Eg (2) Pl (2) |
| 5 | Bc (2) Eg (2) Ca (2) |
| 6 | Pva (1) Eg (2) Pl (2) |
| 7 | Pip (2) Eg (2) |
| 8 | Eg (1) |
| 9 | Pva (1) Pip (1) Eg (1) Pl (1) |
| 10 | Pip (1) |
| 11 | Pvt (2) Bc (2) Pip (1) Eg (1) |
| 12 | Eg (2) Pl (2) |
| 13 | Eg (2) |
| 15 | Pvt (1) Eg (2) |
| 16 | Ca (1) |
| 17 | Bc (1) Eg (2) Ca (2) |
| 18 | Eg (2) Pr (1) Ca (1) |
| 19 | Eg (2) |
| 20 | Pip (1) Eg (2) |
| 21 | Bc (2) Pip (1) Eg (2) |
| 22 | Eg (2) |
| 23 | Eg (1) |
| 24 | Pl (2) Ca (2) |
| 25 | Pva (1) Eg (1) Pl (2) |
| 26 | Pva (1) Eg (2) Pl (2) |
| 27 | Eg (2) Pl (2) |
| 28 | Pl (1) |

Table II (continued)....

| Compound of Example No. | Greater than 50% disease control achieved in the below indicated tests | | |
|---|---|---|---|
| 29 | Pl (2) | | |
| 30 | Pva (2) | Pl (2) | |
| 31 | Pip (2) | Eg (2) | |
| 32 | Pip (1) | Eg (1) | Pl (2) |
| 33 | Pip (1) | Eg (2) | Pl (2) |
| 34 | Eg (2) | | |
| 35 | Pl (2) | | |
| 39 | Pip (1) | Eg (1) | Pl (1) |
| 40 | Eg (2) | Pl (2) | |
| 41 | Eg (2) | Pl (2) | |
| 42 | Pl (2) | | |
| 43 | Pl (1) | | |
| 45 | Pip (1) | Eg (1) | Pl (2) |
| 46 | Eg (2) | Pl (2) | |
| 47 | Eg (2) | Pl (2) | |
| 48 | Eg (2) | Pl (1) | |
| 49 | Bc (1) | Pip (1) | Eg (1) |
| 50 | Pip (1) | Eg (2) | Pl (2) |
| 51 | Eg (1) | | |
| 52 | Eg (2) | Pl (1) | |
| 54 | Eg (1) | Pl (2) | |
| 55 | Eg (1) | Pl (2) | |
| 56 | Eg (1) | Pl (1) | |
| 57 | Eg (1) | Pl (2) | |

K 1863

CLAIMS

1. A fungicidal composition which comprises a carrier and, as active ingredient, a compound of the general formula (I)

$$Ar^1R^1C = CAr^2R^2 \qquad (I)$$

or an N-oxide, salt or metal salt complex thereof, wherein one of $Ar^1$ and $Ar^2$ represents an optionally substituted 6 membered heteroaromatic ring containing 1 or 2 nitrogen atoms and the other of $Ar^1$ and $Ar^2$ represents an optionally substituted 5 or 6 membered heterocyclic ring having 1 or 2 heteroatoms or an optionally substituted phenyl group; $R^1$ represents a hydrogen atom or an optionally substituted alkyl group; and $R^2$ represents a cyano group, a -COOH group or an ester or thioester thereof, or a group

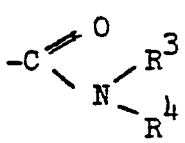 or 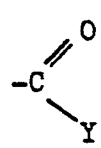

wherein each of $R^3$ and $R^4$ independently represents a hydrogen atom or an optionally substituted alkyl or phenyl group or $R^3$ and $R^4$ together with the interjacent nitrogen atom represent a heterocyclic ring and Y represents a halogen atom.

2. A composition as claimed in claim 1 in which one of $Ar^1$ and $Ar^2$ represents an unsubstituted pyrazine or an unsubstituted pyridyl group and the other of $Ar^1$ and $Ar^2$ represents an unsubstituted pyridyl group or an optionally substituted phenyl group.

3. A composition as claimed in claim 2 in which one of $Ar^1$ and $Ar^2$ represents an unsubstituted 3-pyridyl group and the other of $Ar^1$ and $Ar^2$ represents an unsubstituted 3-pyridyl group or a halo-substituted phenyl group.

4. A composition as claimed in claim 3 in which one of $Ar^1$ and $Ar^2$ represents an unsubstituted 3-pyridyl group and the other of $Ar^1$ and $Ar^2$ represents a dichloro substituted phenyl group.

5. A composition as claimed in any one of the preceding claims in which $R^1$ represents a hydrogen atom.

6. A composition as claimed in any one of the preceding claims in which $R^2$ represents a cyano group, a -COOH group or an ester or thioester thereof or a group

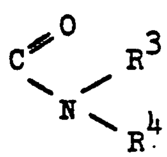

or

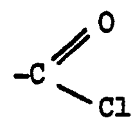

wherein each of $R^3$ and $R^4$ independently represents a hydrogen atom, an unsubstituted alkyl group of up to 6 carbon atoms or an optionally substituted phenyl group, or $R^3$ and $R^4$ together with the interjacent nitrogen atom represent a pyrrolidine or piperidine ring.

7. A composition as claimed in any one of the preceding claims in which the active ingredient is any one of those given in Examples 1 to 38 herein.

8. A composition as claimed in any one of the preceding claims which comprises at least two carriers, at least one of which is a surface-active agent.

9. A compound of the general formula I or an N-oxide, salt or metal salt complex thereof, wherein $Ar^1$, $Ar^2$, $R^1$ and $R^2$ are as defined in any one of claims 1 to 6, with the proviso that when $Ar^1$ is an unsubstituted pyridyl group and $R^1$ is a hydrogen atom, $R^2$ is not a cyano group when $Ar^2$ is an unsubstituted phenyl group or a 2-chloro, 4-chloro, 4-amino, 4-dimethylamino, 4-acetamido, 4-nitro, 4-methoxy or 3,4-dimethoxy substituted phenyl group, $R^2$ is not a $-CONH_2$ group when $Ar^2$ is an unsubstituted phenyl group or a 4-chloro or 4-dimethylamino group, $R^2$ is not a $-COOH$ group when $Ar^2$ is an unsubstituted phenyl group or a 4-nitrophenyl group, and $R^2$ is not a $-COCl$ group when $Ar^2$ is an unsubstituted phenyl group; and further provided that if $Ar^2$ is an unsubstituted pyridyl group and $R^1$ is a hydrogen atom, $R^2$ is not a cyano group if $Ar^1$ is an unsubsituted phenyl group or a 4-dimethylaminophenyl group, and $R^2$ is not a $-COOH$, $-COCl$ or $-CONH_2$ group if $Ar^1$ is an unsubstituted phenyl group.

10. A compound as claimed in claim 9 and named in any one of the Examples 1 to 5 and 8 to 38 herein.

11. A process for the preparation of a compound as claimed in claim 9 which comprises reacting a compound of the general formula (II)

$$Ar^1-\overset{O}{\overset{\|}{C}}-R^1 \qquad (II)$$

with a compound of the general formula III

$$Ar^2-CH_2-R^2 \qquad (III)$$

wherein $Ar^1$, $Ar^2$, $R^1$ and $R^2$ are defined in claim 9; and if desired, converting the resulting compound of the inventon into any other compound of the invention.

12. A process as claimed in claim 11 substantially as hereinbefore described in any one of Examples 1 to 7 herein.

13. A compound as claimed in claim 9 whenever prepared by a process as claimed in either claim 11 or claim 12.

14. A method of controlling fungus at a locus which comprises applying to the locus a compound as claimed in any one of claims 9, 10 and 13, or a composition as claimed in any one of claims 1 to 8.